# EUROPÄISCHE PATENTSCHRIFT

(11) **EP 2 200 768 B1**
(45) Veröffentlichungstag und Bekanntmachung des Hinweises auf die Patenterteilung: **21.10.2015**
(21) Anmeldenummer: 08835906.2
(22) Anmeldetag: 27.08.2008
(51) Int. Cl.: B22F 1/00, B22F 3/15, B22F 3/22, B22F 9/08

(54) **VERFAHREN ZUR HERSTELLUNG VON HALBZEUGE AUS NITI-FORMGEDÄCHTNISLEGIERUNGEN**
METHOD FOR PRODUCING SEMI-FINISHED PRODUCTS FROM NITI SHAPE MEMORY ALLOYS
PROCÉDÉ DE FABRICATION DE SEMI-PRODUITS À PARTIR D'ALLIAGES NiTi À MÉMOIRE DE FORME

(30) Priorität: 04.10.2007 DE 102007047523
(43) Veröffentlichungstag der Anmeldung: 30.06.2010
(73) Patentinhaber: Forschungszentrum Jülich GmbH, 52425 Jülich (DE)
(72) Erfinder: KOEHL, Manuel, 40476 Düsseldorf (DE); BRAM, Martin, 52428 Jülich (DE); COENEN, Berthold, 41836 Hückelhoven (DE); BUCHKREMER, Hans, Peter, 52525 Heinsberg (DE); STOEVER, Detlev, 52382 Niederzier (DE)
(86) Internationale Anmeldenummer: PCT/DE2008/001427
(87) Internationale Veröffentlichungsnummer: WO 2009/043323

(56) Entgegenhaltungen:
- EP-A- 0 045 985
- WO-A-02/085561
- J. MENTS ET AL: "Influence of heat treatments on the mechanical properties of high-quality Ni-rich NiTi produced by powder metallurgical methods" MATERIALS SCIENCE AND ENGINEERING A, Bd. 481-482, 2008, Seiten 630-634, XP002508745
- ZHENG H X ET AL: "Powder metallurgical production of TiNiNb and TiNiCu shape memory alloys by combination of pre-alloyed and elemental powders" JOURNAL OF ALLOYS AND COMPOUNDS, ELSEVIER SEQUOIA, LAUSANNE, CH, Bd. 463, Nr. 1-2, 8. September 2008 (2008-09-08), Seiten 250-256, XP023176014 ISSN: 0925-8388 [gefunden am 2008-07-23]
- C. TRÉPANIER, T.K. LEUNG, M. TABRIZIAN, L'H. YAHIA, J.-G. BIENVENU, J.-F. TANGUAY, D.L. PIRON, L. BILODEAU: "Preliminary investigation of the effects of surface treatments on biological response to shape memory NiTi stents" J. BIOMED. MATER. RES., Bd. 48, 1999, Seiten 165-171, XP002508746

## Beschreibung

Die Erfindung betrifft Halbzeuge aus einer Formgedächtnislegierung auf Nickel-Titan-Basis, und insbesondere deren Herstellung.

### Stand der Technik

Unter Halbzeug wird der Oberbegriff für vorgefertigte Rohmaterialformen, wie beispielsweise Bleche, Stangen, Rohre und Coils verstanden. In der Metallverarbeitung stellen Halbzeuge die mit Abstand verbreitetste Lieferform für Rohmaterialien aus Metall und Kunststoff dar.

Typisch für Halbzeuge ist, dass sie in aller Regel nicht in der ursprünglichen Abmessung bzw. Größe Verwendung finden. Meist besteht der erste Verarbeitungsschritt in einem Zuschnitt, bei dem durch ein geeignetes Trennverfahren (z. B. Sägen) der benötigte Materialabschnitt abgetrennt wird.

Für die Herstellung von Halbzeugen sind insbesondere zwei Fertigungsverfahren bekannt, das Metal Injection Molding (MIM) und das heißisostatische Pressen (HIP). Diese beiden Verfahren weisen jeweils den Vorteil der pulvermetallurgischen Herstellung von Bauteilen bzw. Halbzeugen auf, der im geringen Nachbearbeitungsbedarf der Komponenten besteht.

Das Metal Injection Molding ist ein Verfahren zur Herstellung von metallischen Kleinst- und Kleinteilen. Das Verfahren kombiniert zwei bekannte Herstelltechnologien, die eigentlich wenig miteinander zu tun haben, das Kunststoffspritzgießen und das Sintern von Metallen. Das MIM-Verfahren umfasst vier Teilschritte, die Spritzmassenaufarbeitung, das Spritzgießen, das Bindemittelentfernen und das Sintern. Der erste Schritt besteht in dem Mischen eines geeigneten, sehr feinen Metallpulvers mit einem Binder zu einem spritzfähigen Ausgangsmaterial. Im Gegensatz zum konventionellen Pressen und Sintern, wo üblicherweise Dichten von 90% der theoretischen Werkstoffdichte erzielt werden, erreicht das Metallpulverspritzgussverfahren Dichten zwischen 96 % und 100 % der theoretischen Werkstoffdichte. Dadurch werden vorteilhaft Materialeigenschaften erreicht, die weitgehend jenen des aus dem Vollen gearbeiteten Teils entsprechen.

Auf der anderen Seite ist es über das abgewandelte MIM-Verfahren mit Einsatz von Platzhaltern möglich auch besonders poröse Halbzeuge herzustellen. Die Porosität kann dabei vorteilhaft zwischen 10 und 80 Vol.-% eingestellt werden.

Da die über das MIM-Verfahren hergestellten Bauteile bzw. Halbzeuge in der Regel nicht mechanisch nachbearbeitet werden müssen, bietet das Metal Injection Molding umso größere Vorteile, je schwieriger ein Material üblicherweise bearbeitbar ist. Daher ist dieses Verfahren, insbesondere für rostfreie Stähle, weichmagnetische Legierungen, Eisen/Nickel-Werkstoffe, aber auch Werkzeugstähle und Sonderlegierungen, wie beispielsweise Formgedächtnislegierungen auf Nickel-Titan-Basis geeignet.

Formgedächtnislegierungen sind Metalle, die nach einer Verformung wieder ihre ursprüngliche Gestalt annehmen, wenn man sie auf eine bestimmte Temperatur erwärmt. Dabei können sie beachtliche Kräfte entfalten. Mögliche Einsatzgebiete für Formgedächtnislegierungen sind Mikromanipulatoren und Roboteraktuatoren, die die fließenden Bewegungen menschlicher Muskeln nachahmen können. Bei Stahlbetonstrukturen könnten Sensoren aus Formgedächtnislegierungen eingesetzt werden, die beispielsweise Risse im Beton oder Korrosion in den Stahl-Verstärkungsstangen aufspüren und den im Innern auftretenden Spannungen entgegenwirken.

Bisher werden Formgedächtnislegierungen auf der Basis der intermetallischen Phase NiTi bevorzugt schmelzmetallurgisch hergestellt. Die konventionelle Formgebung durch mechanische Bearbeitung von schmelzmetallurgischen Halbzeugen aus NiTi-Werkstoffen ist jedoch begrenzt, da die Legierungen im martensitischen Zustand eine hohe Bruchdehnung aufweisen und damit eine schlechte Zerspannbarkeit besitzen. Infolgedessen ist eine Nachbearbeitung von schmelzmetallurgisch hergestellten Bauteilen nur unter zeitlich hohem Aufwand und hohem Werkzeugverschleiß möglich.

Der Einsatz des Metallformspritz-Verfahrens auch für NiTi-Formgedächtnislegierungen scheiterte bislang häufig daran, dass neben einer wirtschaftlichen Produktion auch alle für die Formgedächtniseigenschaften notwendigen geringen Verunreinigungsgehalte im Endprodukt gewährleistet sein müssen. Die sich ergebende Schwierigkeit bei der NiTi-Bauteilfertigung über Metallpulverspritzguß (MIM) liegt darin, den Gehalt an Sauerstoff und Kohlenstoff so gering wie möglich zu halten. Hohe Verunreinigungsgehalte führen zu einer verminderten Sinteraktivität der Metallpulver und verschlechtern die Werkstoffeigenschaften im gesinterten Bauteil (z. B. durch Versprödung). Ferner ist eine weitere wichtige Voraussetzung für die Herstellung von NiTi-Formgedächtnislegierungen die genaue und reproduzierbare Einstellung der Legierungszusammensetzung. Bereits geringe Abweichungen in der Zusammensetzung führen zu deutlichen Abweichungen der charakteristischen Eigenschaften (z.B. der Umwandlungstemperaturen).

Bei der Pulverherstellung entstehen in der Regel Partikel mit einer mehr oder weniger breiten Partikelgrößenverteilung, die auch einen Anteil an gröberen Partikeln aufweist. Bei der Pulverherstellung über Gasverdüsen ist bekannt, dass derzeit schon mittlere Partikelgrößen d₅₀ von ca. 50 µm oder noch kleiner hergestellt werden können.

Aus J. Mentz et al.: "Influence of heat treatments on the mechanical properties of high-quality Ni-rich NiTi produced by powder metallurgical methods, Materials Science and Engineering: A, Bd.481-482, 2008, Seiten 630-634 ist bekannt, dass legiertes NiTi-Pulver mit einem Anteil von 50,6 at.-% Ni durch Gasatomisierung hergestellt werden kann. Dieses Pulver wurde durch Sieben in zwei Fraktionen getrennt, so dass eine Grobfraktion mit d₅₀ = 67 Mikrometern und eine Feinfraktion mit d₅₀ = 22 Mikrometern erhalten wurde. Die Grobfraktion wurde vorteilhaft in einem HP-Prozess eingesetzt, während die Feinfraktion in einem MIM-Prozess verwendet wurde.

Die prinzipielle Herstellung von TiNiNb and TiNiCu als Gedächtnislegierungen ist bereits in H. X. Zheng et al.: Powder metallurgical production of TiNiNb and TiNiCu shape memory alloys by combination of pre-alloyed and elemental powders,,,Journal of Alloys and Compounds, Bd. 463, Nr. 1-2, 8 September 2008, Seiten 250-256 beschrieben.

Ferner wird in C. Trépanier, T-.K. Leung, M. Tabrizian, L.H.Yahia, J.-G. Bienvenue, J.-F. Tanguay, D. L. Piron, L. Bilodeau "Preliminary Investigation of the Effects of Surface Treatments on Biological Response to Shape memory NiTi Stents", J. Biomed. Mater. Res., Bd. 48, 1999, Seiten 165-171 die Verwendung von NiTi-Formgedächtnislegierungen für medizinische Produkte, insbesondere stents, offenbart.

Der Einsatz von besonders feinem Ausgangspulver weist zwar verfahrenstechnisch den Vorteil auf, dass diese eine sehr gute Verspritzbarkeit zeigen, bringen in der Regel aber auch die Gefahr einer höheren Verunreinigung über das verwendete Tiegelmaterial mit sich, was sich insbesondere für NiTi-Formgedächtnislegierungen mit einem dritten Legierungszusatz als besonders nachteilig herausgestellt hat.

Für besonders reine und dennoch verunreinigungsarme Pulver, wie sie auch bei der Herstellung von Halbzeugen aus einer Formgedächtnislegierung benötigt werden, bietet sich daher die Pulverherstellung über das tiegelfreie (verunreinigungsfreie) Schmelzen über Induktion und Verdüsen nach dem EIGA (Electrode Induction melting Gas Atomization) Prozess an. Nachteilig hierbei macht jedoch die Partikelfraktion des so hergestellten Pulvers mit einem Partikeldurchmesser größer als 45 µm regelmäßig mehr als 65 Gew.-% aus. Die Abtrennung der benötigten Feinfraktion und die bislang fehlende Verwertung der abgetrennten Grobfraktion machten somit den Einsatz dieses Materials für die Herstellung von Halbzeugen über das MIM-Verfahren sehr teuer.

Für Anwendungen, bei denen besonders feine Partikel notwendig sind, können diese häufig nur über eine Abtrennung des Grobanteils aus einem hergestellten Pulver erhalten werden. So sind beispielsweise Trennschnitte von d₉₇ zwischen 2 und 120 µm durch den Einsatz von Feinsichtem erzielbar.

Das zweite Verfahren zur Herstellung von Halbzeugen ist das heißisostatische Pressen (HIP). Dies ist eine Entwicklung in der Fertigungstechnik, bei welcher Pulver und Feststoffe, besonders Keramiken und Metalle, gleichzeitig heiß gepresst und gesintert werden. Beim heißisostatischen Pressen (HIP) werden unter Anwendung hoher Temperaturen (kleiner als die Materialschmelztemperatur) und einer isostatischen Druckbeaufschlagung von mehreren hundert MPa pulverförmige oder poröse Werkstoffe ganz ohne Binder gleichmäßig verdichtet. Zum Einsatz kommen dabei beispielsweise aus dem Schmelzbad feinst verdüste Materialien, die für qualitativ hochwertige Gefügestrukturen sorgen, wobei sich eine große Gestaltungsfreiheit in der Legierungsauswahl ergibt. Als Druckübertragungsmedium werden beim heißisostatischen Pressen inerte Gase eingesetzt. Pulver oder hochporöse Werkstoffe werden dabei gekapselt, während Bauteile mit geschlossener Porosität auch ungekapselt heißisostatisch verdichtet werden können. Das Ergebnis sind homogene Gefüge, wie sie mit anderen Verfahren kaum zu erreichen sind. Bauteile, die über ein HIP-Verfahren hergestellt worden sind, sind extrem dicht (bis 100 % der theoretischen Dichte) und haben isotrope Eigenschaften. Vorteilhaft kann bei diesem Verfahren auch ein Verbundbauteil aus unterschiedlichen Materialien realisiert werden.

Ein großer Nachteil dieser Technik ist, dass sie sehr aufwändig ist und somit mit sehr hohen Fertigungskosten verbunden ist, besonders bei geringer Maßtoleranz. Als Hauptanwendungsgebiet ist das Nachverdichten von gesinterten, metallischen und keramischen Werkstücken für die Luft- und Raumfahrt, die Automobilindustrie oder medizinische Implantate zu nennen.

### Aufgabe und Lösung

Der Erfindung liegt die Aufgabe zugrunde, kostengünstig Halbzeuge, insbesondere Halbzeuge aus einer Formgedächtnislegierung zu schaffen, sowie ein kostengünstiges Verfahren zur Herstellung dieser Halbzeuge bereit zu stellen.

Die Aufgaben der Erfindung werden gelöst durch ein Verfahren zur Herstellung von Halbzeugen gemäß Hauptanspruch. Vorteilhafte Ausgestaltungen des Verfahrens ergeben sich aus den jeweils rückbezogenen Ansprüchen.

### Gegenstand der Erfindung

Die Erfindung basiert auf dem Gedanken, dass das Verfahren zur Herstellung von Halbzeugen über das heißisostatische Pressen dahingehend kostentechnisch verbessert werden kann, dass als Ausgangspulver gerade der Anteil an Partikeln eingesetzt wird, der üblicherweise bei der Herstellung von Pulvern für ein MIM-Verfahren anfällt, aber dort gerade nicht eingesetzt wird. Während sich die verwendete Pulvergröße, insbesondere der gröbere Anteil, bei dem Verfahren des heißisostatischen Pressens (HIP) qualitativ nahezu gar nicht auswirkt, ist der Einsatz des besonders feinen Partikelanteils für den MIN-Prozess von großem Vorteil.

Das bedeutet, dass durch geschickte Kombination von zwei Verfahren, dem des metal injection molding (MIM)-Verfahrens auf der einen Seite und dem des heißisostatischen Pressens (HIP) für die Herstellung eines Halbzeugs auf der anderen Seite, eine besonders vorteilhafte Kosteneffizienz erreicht werden kann, indem beide Verfahren jeweils auf unterschiedliche Anteile eines Herstellungsprozesses für Pulver zurückgreifen.

Die Pulverherstellung über ein an sich aufwendiges Verfahren rechnet sich dann trotzdem, wenn nach der Abtrennung des für das MIM-Verfahren benötigte Feinanteil, der Grobanteil sinnvoll weiter verwendet werden kann, in diesem Fall zur Herstellung von Halbzeugen über das HIP-Verfahren. Der Einsatz des abgetrennten gröberen Pulvers bei dem HIP-Verfahren stellt kaum einen Nachteil dar, da sich das Pulver vor der Druckbeaufschlagung dennoch gut verfestigen lässt, und sich Parameter zur Druckbeaufschlagung nicht nennenswert von denen unterscheiden, die für die Herstellung des Halbzeugs aus einer normal verteilten Pulverfraktion eingestellt werden würden.

Bei der Pulverherstellung von hochreaktiven Metallen und Legierungen hat sich das verunreinigungsfreie Schmelzen und Verdüsen (EIGA) als besonders geeignetes Verfahren herausgestellt. Zur Charakterisierung von Pulvern wird insbesondere die Korngröße, bzw. die Korngrößenverteilung herangezogen. Die Korngrößenverteilung ist das Ergebnis einer Korngrößenanalyse. Sie stellt letztlich nichts anderes als eine Häufigkeitsverteilung in Form eines Balkendiagramms dar. Gegen den klassierten Äquivalentdurchmesser, alternativ der Korngröße (Abszisse) wird der prozentuale Volumenanteil der Körner aufgetragen. Die Korngrößenverteilung wird auch gerne als Summenkurve dargestellt.

Praktisch wird die Korngrößenverteilung meist durch Siebung (Sieblinie), Sedimentation oder optische Verfahren ermittelt. Es gibt eine Vielzahl weiterer Verfahren, die auf bestimmte Stoffe oder Größenbereiche beschränkt sind. In manchen Zusammenhängen werden disperse Systeme durch Mittelwerte gekennzeichnet.

Mit Hilfe des EIGA-Verfahrens sind in der Regel Pulver mit einem mittleren Äquivalenzdurchmesser d₅₀ von ca. 80 µm herstellbar. Für den nachfolgenden Einsatz dieser Pulver bei einem MIM-Prozess wird jedoch häufig nur der Feinanteil einer solchen Pulvermischung, z. B. Pulver kleiner als 45 µm eingesetzt, vorteilhaft sogar häufig nur kleiner als 25 µm. Der Grobanteil mit einer mittleren Korngröße größer als 45 µm, der teilweise mehr als 65 Gew.-% der hergestellten Pulvermischung ausmacht, wird häufig nicht genutzt, führt aber dazu, dass dieses Verfahren insgesamt recht teuer ist.

Im Rahmen dieser Erfindung wurde herausgefunden, dass gerade dieser Grobanteil bestens dazu genutzt werden kann, um in einem HIP-Verfahren zur Herstellung von Halbzeugen eingesetzt zu werden. Bislang werden bei der Umformung des heißisostatischen Pressens Pulvermischungen mit einer entsprechenden Korngrößenverteilung eingesetzt, das heißt so, wie sie bei der entsprechenden Pulverherstellung anfallen.

Erfindungsgemäß wird nunmehr zunächst ein nahezu verunreinigungsfreies Pulver aus einer Nickel-Titan Basislegierung erzeugt. Als Herstellungsverfahren für dieses Material und die entsprechenden Korngrößenverteilung bietet sich beispielsweise die Gasverdüsung (EIGA) an. Dabei könnte beispielsweise das Material in der Regel mit einer entsprechenden Korngrößenverteilung hergestellt werden, wobei der Anteil mit einer mittleren Korngröße größer als 45 µm einen Gewichtsanteil von mehr als 65 % ausmacht.

In einem weiteren Schritt wird der Feinanteil des Materials unterhalb einer bestimmten Trenngrenze T1 abgetrennt. Ferner wird das Grobpulver einem weiteren Trennschnitt T2 > T1 im Bereich von 50 bis 150 µm unterzogen, wobei nur der Grobanteil der Pulvermischung mit einer mittleren Korngröße zwischen T1 und T2 für die Herstellung von Halbzeugen über das HIP-Verfahren verwendet wird. Der Feinanteil wird vorteilhaft für einen MIM-Prozess eingesetzt. Die Trennschnitte selbst können über herkömmliche Verfahren realisiert werden, wobei ein Trennschnitt T1 im Bereich zwischen 1 und 100 µm, insbesondere zwischen 10 und 50 µm für das erfindungsgemäße Verfahren als sinnvoll betrachtet wird.

Als für das Verfahren geeignete Formgedächtnislegierungen sind insbesondere die NiTi-Formgedächtnislegierungen zu nennen. Dabei können sowohl die reinen NiTi-Legierungen eingesetzt werden, als auch ternäre oder quaternäre Legierungen auf Basis von NiTi. Diese ternären oder quaternären Legierungen weisen neben NiTi als Basis geringe Mengen an wenigstens einem weiteren Legierungselement X aus der Gruppe (X = Cu, Nd, Hf, Zr, Pt oder Pd) auf. In der Literatur werden dazu beispielsweise die folgenden Größenordnungen der Beimischung offenbart: Für Nb zwischen 4,5 und 9 at.-%, für Cu zwischen 5 und 20 at.-%, für Hf zwischen 9,5 und 20 at.-%, für Zr zwischen 1 und 50 at.-% und für Pt bis maximal 10 at.%.

Die Vorteile der Erfindung können wie folgt zusammengefasst werden:
- Das MIM-Verfahren zur Herstellung von Halbzeugen aus einer Formgedächtnislegierung wird rentabler und ist kostengünstiger zu realisieren.
- Die dadurch erzeugten Halbzeuge weisen eine pulvermetallurgische Mikrostruktur auf, insbesondere
   ∘ sind die Legierungselemente besonders homogen verteilt,
   ∘ entstehen regelmäßig keine Gussfehler oder Seigerungen,
   ∘ entsteht keine Anisotropie des Gefüges infolge von Verarbeitungsprozessen,
   ∘ ist eine Verarbeitung von ternären Legierungen möglich, die auf Grund ihrer mechanischen Eigenschaften nicht durch konventionelle Formgebung bearbeitet werden können.

### Spezieller Beschreibungsteil

Nachfolgend wird die Erfindung anhand eines Ausführungsbeispiels für die Herstellung eines Halbzeugs mit Hilfe des HIP-Verfahrens näher erläutert, ohne dass dadurch der Schutzbereich eingeschränkt wird.

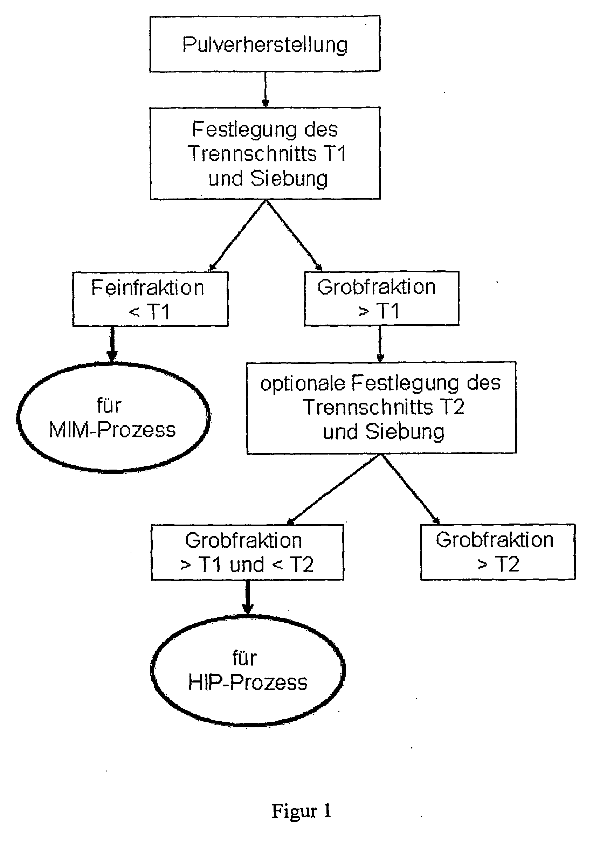

Das prinzipielle Verfahren wird aus dem Schema der Figur 1 deutlich.

Es wurde ein Pulver aus NiTi-Formgedächtnislegierung hergestellt, wobei von der gesamten Charge vier Fraktionen: a) < 25 µm, b) 25 bis 45 µm, c) 45 - 100 µm und d) > 100 µm abgesiebt wurden. Dabei betrug die Ausbeute an der Fraktion a) + b), also der Teilchen mit einer mittleren Korndurchmesser von kleiner 45 µm, ca. 35 Gew.-%.

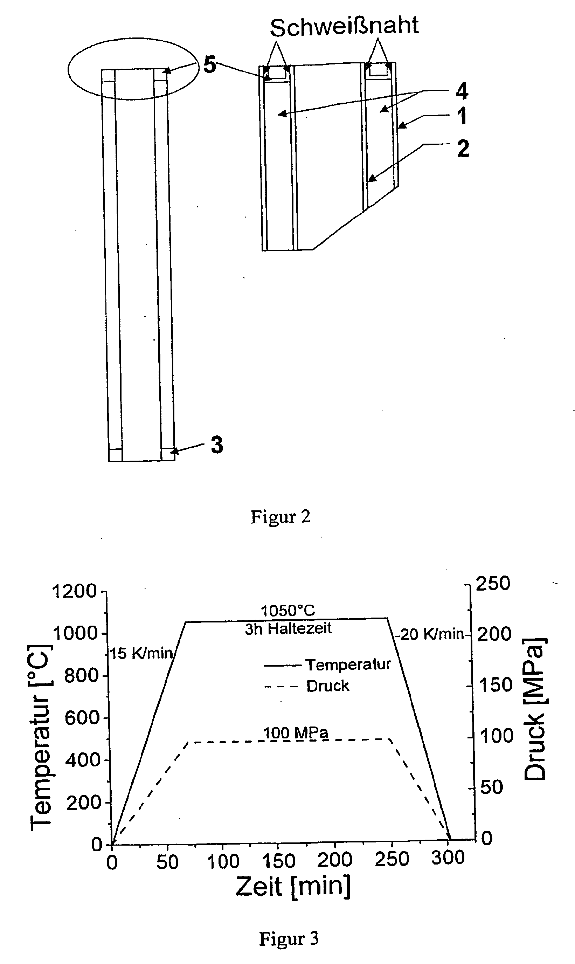

Die Grobfraktion c) aus nahezu sphärischen Pulvern mit einem mittleren Durchmesser größer 45 µm und kleiner 100 µm wurde in eine HIP Kapsel überführt, deren Geometrie aus der Figur 2 zu entnehmen ist. Die Fraktion d) größer als 100 µm wurde dabei außer Acht gelassen, da diese regelmäßig auch sehr große Grobpartikel, so genannte Flakes aufweist. Bei der HIP-Kapsel handelte es sich um ein Außenrohr (1) mit einem darin zentrisch angeordneten Innenrohr (2). Beide Rohre sind über einen unteren Deckel (3) fixiert und verschweißt.

Das Pulver (4) wurde zwischen Innen- und Außenrohr eingefüllt und verdichtet, um eine möglichst hohe Klopfdichte zu erreichen. Dazu wurde die zunächst locker gefüllte Kapsel mit Aufsatz auf einem Exzenter getriebenen Tisch vertikalen Fallstößen geringer Frequenz ausgesetzt. Dieses als Stampfvolumeter bekannte Gerät wird üblicherweise zur Ermittlung eines Volumens und nach einer Verdichtung eingesetzt, sollte aber in diesem Fall lediglich die Schüttung auf die Klopfdichte verdichten. Der Grad der Verdichtung hängt von der Elastizität der Unterlage und der Fallhöhe (Fallverzögerung) sowie der Zahl der Fallstöße ab. Anschließend wurde der obere Deckel (5) der HIP-Kapsel durch Elektronenstrahlschweißen im Vakuum, bzw. nach der Evakuierung der Kapsel verschweißt.

Das Rohr mit dem so vorverdichteten NiTi wurde hängend einem definierten Druck und Temperaturprofil (HIP-Zyklus) unterworfen, welches aus der Figur 3 ersichtlich wird. Die Aufhängung der HIP-Kapseln bewirkte vorteilhaft eine höhere Formhaltigkeit der Proben.

Alternativ zum vorgenannten Versuchsaufbau gemäß der Figur 2 kann das Innenrohr zur höheren Formstabilität des NiTi oder NiTi-X während des HIP-Prozesses auch aus Vollmaterial sein.

Nachfolgend fand das Abdrehen bzw. das Ausbohren der Edelstahlkappe der Kapsel und das Rohrziehen statt, um so das erfindungsgemäße Halbzeug zu erhalten.

In weiteren Schritten kann dann aus diesem Halbzeug beispielsweise die konventionelle Weiterverarbeitung zu Stents oder Klenunringen erfolgen, wobei als Material für die Klemmringe insbesondere die bislang schlecht bearbeitbaren Formgedächtnislegierungen (NiTi-X, X = Cu, Nb, Hf, Zr, Pt, Pd) interessant sind.

## Patentansprüche

1. Verfahren zur Herstellung von rohrförmigen Halbzeugen aus einer Formgedächtnislegierung zur Stentherstellung mit den Schritten
a) es wird ein Pulver über eine Gasverdüsung aus einer Formgedächtnislegierung hergestellt,
b) das Pulver wird bei einem ersten Trennschnitt T1 im Bereich von 1 und 100 µm in eine Grobfraktion mit einer mittleren Korngröße > T1 und eine Feinfraktion mit einer mittleren Korngröße < T1 aufgeteilt,
c) die Grobfraktion wird einem weiteren Trennschnitt T2 > T1 im Bereich von 50 bis 150 µm unterzogen,
d) die Feinfraktion wird für die Herstellung eines ersten Halbzeuges unter Einsatzes des Metal Injection Molding (MIM)-Verfahrens eingesetzt,
e) nur das Pulver der Grobfraktion mit einer mittleren Korngröße zwischen T1 und T2 wird für die Herstellung des rohrförmigen zweiten Halbzeuges unter Einsatzes des heiss isostatischen Pressens (HIP) eingesetzt.

2. Verfahren nach Anspruch 1, bei dem das Pulver über das EIGA (electrode induction melting gas atomisation) Verfahren hergestellt wird.

3. Verfahren nach einem der Ansprüche 1 bis 2, bei dem der erste Trennschnitt T1 im Bereich von 10 bis 50 µm durchgeführt wird.

4. Verfahren nach einem der Ansprüche 1 bis 3, bei dem eine Formgedächtnislegierung aus NiTi eingesetzt wird, mit einem Verhältnis von Ni zu Ti von 45 at.% zu 55 at.% bis 55 at.% zu 45 at.%, insbesondere mit einem Verhältnis von 48,5 at.% zu 51,5 at.% bis 51 at.% zu 49 at.%.

5. Verfahren nach Anspruch 4, bei dem die eingesetzte Formgedächtnislegierung eine dritte Komponente X aus der Gruppe (X = Cu, Nd, Hf, Zr, Pt oder Pd) aufweist, wobei der Anteil an Cu zwischen 5 und 20 at.-%, der Anteil an Nd zwischen 4,5 und 9 at.-%, der Anteil an Hf zwischen 9,5 und 20 at.-%, der Anteil an Zr zwischen 1 und 50 at.-% und der Anteil an Pt bis maximal 10 at.-% beträgt.

## Claims

1. Method for producing tubular semi-finished products from a shape memory alloy for the production of stents comprising the steps
a) a powder is produced from a shape memory alloy by gas atomisation,
b) in a first separating cut T1 in the range between 1 and 100 µm, the powder is separated into a coarse fraction with an average grain size > T1 and a fine fraction with an average grain size < T1,
c) the coarse fraction is subjected to a further separating cut T2 > T1 in the range from 50 to 150 µm,
d) the fine fraction is used for the manufacture of a first semi-finished product using the metal injection moulding (MIM) method,
e) only the powder of the coarse fraction with an average grain size of between T1 and T2 is used for the manufacture of the second, tubular, semi-finished product using hot isostatic pressing (HIP).

2. Method according to claim 1, wherein the powder is produced using the EIGA (electrode induction melting gas atomisation) method.

3. Method according to one of the claims 1 to 2, wherein the first separating cut T1 is carried out in the range from 10 to 50 µm.

4. Method according to one of the claims 1 to 3, wherein a NiTi shape memory alloy is used, with a ratio of Ni to Ti from 45 at.% to 55 at.% up to 55 at.% to 45 at.%, in particular with a ratio from 48.5 at.% to 51.5 at.% up to 51 at.% to 49 at.%.

5. Method according to claim 4, wherein the shape memory alloy which is used has a third component X from the group (X = Cu, Nd, Hf, Zr, Pt or Pd), wherein the content of Cu is between 5 and 20 at.%, the content of Nd between 4.5 and 9 at.%, the content of Hf between 9.5 and 20 at.%, the content of Zr between 1 and 50 at.% and the content of Pt up to a maximum of 10 at.%.

## Revendications

1. Procédé servant à fabriquer des produits semi-finis tubulaires à partir d'un alliage à mémoire de forme pour la fabrication d'endoprothèses, avec les étapes suivantes :
a) une poudre est fabriquée par une atomisation gazeuse à partir d'un alliage à mémoire de forme ;
b) la poudre est divisée par une première coupe de séparation T1 dans la plage comprise entre 1 et 100 µm, en une fraction à gros grains avec une taille de grain moyenne > T1 et en une fraction à grains fins avec une taille de grain moyenne < T1 ;
c) la fraction à gros grains est soumise à une autre coupe de séparation T2 > T1 dans la plage allant de 50 à 150 µm ;
d) la fraction à grains fins est utilisée pour la fabrication d'un premier produit semi-fini en utilisant le procédé MIM (Metal Injection Molding ou moulage par injection de métal) ;
e) la poudre de la fraction à gros grains avec une taille de grain moyenne comprise entre T1 et T2 n'utilisée que pour la fabrication du deuxième produit semi-fini tubulaire en utilisant le procédé de pressage isostatique à chaud (HIP).

2. Procédé selon la revendication 1, dans le cadre duquel la poudre est fabriquée par l'intermédiaire du procédé EIGA (electrode induction melting gas atomisation ou atomisation gazeuse à fusion par induction d'électrode).

3. Procédé selon l'une quelconque des revendications 1 à 2, dans le cadre duquel la première coupe de séparation T1 est effectuée dans la plage allant de 10 à 50 µm.

4. Procédé selon l'une quelconque des revendications 1 à 3, dans le cadre duquel un alliage à mémoire de forme en NiTi est utilisé, avec un rapport entre Ni et Ti allant de 45 % at.:55 % at. à 55 % at.: 45 % at., en particulier un rapport allant de 48,5 % at.: 51,5 % at. à 51 % at.:49 % at.

5. Procédé selon la revendication 4, dans le cadre duquel l'alliage à mémoire de forme utilisé présente un troisième composant X issu du groupe (X = Cu, Nd, Hf, Zr, Pt ou Pd), sachant que la proportion en Cu est comprise entre 5 et 20 % at., que la proportion en Nd est comprise entre 4,5 et 9 % at., que la proportion en Hf est comprise entre 9,5 et 20 % at., que la proportion en Zr est comprise entre 1 et 50 % at. et que la proportion en Pt peut aller jusqu'à 10 % at. au maximum.
